(19) Europäisches Patentamt
European Patent Office
Office européen des brevets

(11) Veröffentlichungsnummer: **0 131 776**
**B1**

(12) # EUROPÄISCHE PATENTSCHRIFT

(45) Veröffentlichungstag der Patentschrifft: **28.02.90**

(51) Int. Cl.⁵: **C 07 D 277/80**

(21) Anmeldenummer: **84106998.2**

(22) Anmeldetag: **19.06.84**

(54) **Verfahren zur Herstellung von Thiazolyl-2-sulfenamiden.**

(30) Priorität: **16.07.83 DE 3325724**

(43) Veröffentlichungstag der Anmeldung:
**23.01.85 Patentblatt 85/04**

(45) Bekanntmachung des Hinweises auf die Patenterteilung:
**28.02.90 Patentblatt 90/09**

(84) Bennante Vertragsstaaten:
**DE FR GB IT**

(56) Entgegenhaltungen:
**EP-A-0 029 718**
**US-A-3 022 300**
**US-A-4 182 873**

**Die Akte enthält technische Angaben, die nach dem Eingang der Anmeldung eingereicht wurden und die nicht in dieser Patentschrift enthalten sind.**

(73) Patentinhaber: **Akzo Patente GmbH**
**Postfach 10 01 49 Kasinostrasse 19-23**
**D-5600 Wuppertal-1 (DE)**

(72) Erfinder: **Zengel, Hans, Dr., Dipl.-Ing.**
**Nordring 6**
**D-8751 Kleinwallstadt (DE)**
Erfinder: **Eisenhuth, Ludwig, Dr., Dipl-Chem.**
**Lautenhofstrasse 44**
**D-8753 Obernburg-Eisenbach (DE)**
Erfinder: **Bergfeld, Manfred, Dr., Dipl.-Chem.**
**August-Pfeffer-Strasse 6**
**D-8765 Erlenbach (DE)**

**EP 0 131 776 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBERGRAF, STOCKHOLM 1990

**Beschreibung**

Die vorliegende Erfindung betrifft ein Verfahren zur Herstellung von Benzthiazolyl-2-sulfenamiden aus 2-Mercaptobenzthiazolen oder Dibenzthiazolyl-2,2'-disulfiden und primären oder sekundären Aminen in Gegenwart von Sauerstoff und einem kupferhaltigen Katalysator in einem Lösungsmittel.

Sulfenamide werden in der Technik in großen Mengen als Vulkanisationsbeschleuniger benötigt.

Es sind bereits mehrere Verfahren zur Herstellung von Sulfenamiden bekannt. Technisch werden sie durch oxydative Kondensation von 2-Mercaptothiazolen mit einem Amin hergestellt, wobei als Oxydationsmittel beispielsweise Chlor, Jod oder Hypochlorit, Wasserstoffperoxide oder andere Peroxide eingesetzt werden (E.L. Carr, J. Org. Chem. 14 921 (1948), US-PS-2 191 657, US-PS-2 417 989, US-PS-2 419 283, US-PS-3 144 652, FP-841 753, GB-PS-655 668 und DE-OS-3 127 193). Weiterhin ist es bekannt, Sulfenamide durch Ersatz des Amids eines N-substituierten Thiazolyl-2-sulfenamids durch ein anderes Amid (DE-OS-1 941 884), durch Ammonolyse von Dithiazolyldisulfid (GB-PS-377 370, US-PS-2 100 692, US-PS-2 214 460 und US-PS-2 226 767) sowie durch elektrochemische Oxydation (DE-OS-2 744 423; J. Org. Chem. 43 (16), 3223 (1978)) herzustellen.

Es ist auch bereits bekannt, bei der oxydativen Umsetzung von 2-Mercaptothiazolen oder Dithiazolyl-2,2'-disulfiden mit einem Amin Sauerstoff als Oxydationsmittel einzusetzen. Bei den Verfahren der CA-PS-863 531, US-PS-3 737 431 und Eu-Patentanmeldung 29 718 werden hierbei Metallphthalocyanine als Katalysator eingesetzt. Die industrielle Herstellung und Handhabung derartiger Katalysatoren ist jedoch problematisch; sie sind empfindlich und teuer. Außerdem werden bei den Verfahren gemäß dieser drei Druckschriften mäßige bis schlechte Ausbeuten erzielt oder sind aufwendige Verfahrensschritte zur Produkt-Isolierung erforderlich.

Aus den DE-OS-2 349 934 und 2 356 686, mit denen im wesentlichen die US-PS-4 182 873 korrespondiert, ist es auch bereits bekannt, die Umsetzung von 2-Mercaptothiazolen bzw. Dithiazolyl-2,2'-disulfiden mit Aminen in Gegenwart von Sauerstoff und Kupfer oder einem anderen seiner Derivate als die Kupferphthalocyaninkomplexe durchzuführen. Vorzugsweise werden hierbei Temperaturen im Bereich von 0 bis 200°C und Sauerstoffpartialdrücke zwischen 0,1 und 30 bar angewendet. Außerdem soll die Umsetzung vorzugsweise in Wasser oder einem organischen Lösungsmittel durchgeführt werden, wobei insbesondere der Überschuß des zur Umsetzung mit dem 2-Mercaptobenzthiazol eingesetzten Amin als Lösungsmittel dienen soll. Wie die Versuchsbeispiele dieser beiden deutschen Offenlegungsschriften zeigen, werden gemäß diesen vorbeschriebenen Verfahren jedoch nur relativ niedrige Ausbeuten an Sulfenamid erhalten. Dies ist, wie weitere Untersuchungen gezeigt haben, ganz offensichtlich darauf zurückzuführen, daß das entstandene Sulfenamid unter den angegebenen Bedingungen in beträchtlichem Ausmaß zu Nebenprodukten (z. B. Benzthiazolylsulfonsäuren, Benzthiazol, Sulfate) weiteroxydiert wird. Bei diesem Verfahren geht also ein Teil des wertvollen Endproduktes verloren. Die bei diesem Verfahren erzielbaren Selektivitäten sind daher für eine wirtschaftliche Anwendung völlig unzureichend.

Es bestand also das Bedürfnis, die Nachteile der bekannten Verfahren zu überwinden. Aufgabe der vorliegenden Erfindung war es daher, ein Verfahren zur Verfügung zu stellen, nach welchem die 2-Mercaptobenzthiazole bzw. Dibenzthiazolyl-2,2'-disulfide mit Aminen und Sauerstoff mit hoher Reaktionsgeschwindigkeit und in hoher Ausbeute und Selektivität in Sulfenamide überführt werden können.

Diese Aufgabe wurde nun gelöst durch ein Verfahren zur Herstellung von Benzthiazolylsulfenamiden der allgemeinen Formel

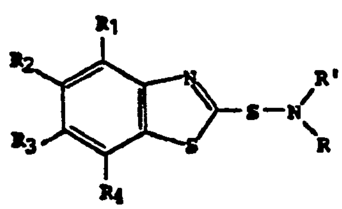

(I)

in der

$R_1$, $R_2$, $R_3$ und $R_4$ gleich oder verschieden sein können und jeweils für Wasserstoff, Chlor, eine Nitrogruppe, eine Hydroxylgruppe, einen Alkyl- oder Alkoxylrest mit 1 bis 6 Kohlenstoffatomen, einen Cycloalkyl- oder Arylrest mit 6 bis 12 Kohlenstoffatomen oder für andere inerte Substituenten stehen, und in der

R und R' gleich oder verschieden sein können und für eine geradkettige, verzweigte oder cyclische Alkylgruppe stehen, wobei R und R' über ein Heteroatom und/oder Kohlenstoffatome miteinander verbunden sein können, so daß sie mit dem Stickstoff eine heterocyclische Gruppierung mit einem oder mehreren Heteroatomen bilden, oder in der R für einen Wasserstoffrest und R' für eine geradkettige verzweigte oder cyclische Alkylgruppe mit 3 bis 8 Kohlenstoffatomen stehen,

durch Umsetzen eines 2-Mercaptobenzthiazols oder Dibenzthiazolyl-2,2'-disulfids der allgemeinen Formel

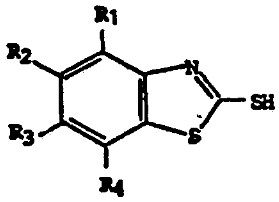

(II)

bzw. 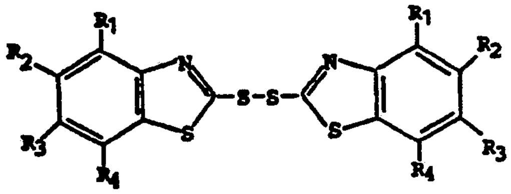

(III)

in der $R_1$, $R_2$, $R_3$ und $R_4$ die oben angegebene Bedeutung haben, mit einem primären oder sekundären Amin der allgemeinen Formel

R
|
HN
|
R'

(IV)

in der R und R' ebenfalls die oben angegebene Bedeutung haben, in Gegenwart von Sauerstoff oder eines sauerstoffhaltigen Gases und von Kupfer oder einem Kupferderivat bei Temperaturen von 0 bis 100°C, das dadurch gekennzeichnet ist, daß man die Umsetzung in Gegenwart von Ammoniak durchführt und als Reaktionsmedium einen Überschuß des oben genannten primären oder sekundären Amins oder eine Mischung dieses überschüssigen Amins mit Wasser und/oder mit einem wassermischbaren organischen Lösungsmittel einsetzt.

Die Substituenten $R_1$, $R_2$, $R_3$ und $R_4$ der Formeln I bis III sind vorzugsweise ein Chloratom, eine Hydroxylgruppe, eine Nitrogruppe, ein geradkettiger oder verzweigtkettiger Alkylrest mit 1 bis 4 Kohlenstoffatomen wie Methyl-, Äthyl-, Propyl-, Isopropyl-, Butyl- oder tert.-Butyl-, ein Alkoxyrest mit 1 bis 4 Kohlenstoffatomen wie Methoxy-, Äthoxy-, Propoxy- oder Butoxy- oder ein Phenyl-, Tolyl-, Äthylphenyl-, Nitrophenyl-, Chlorphenyl- oder Naphthylrest.

Das erfindungsgemäße Verfahren ist insbesondere für die Herstellung von Sulfenamiden des 2-Mercaptobenzthiazols, des wichtigsten Vertreters der 2-Mercaptothiazole, geeignet. Beispiele anderer 2-Mercaptothiazole, welche sich als Ausgangsstoffe für die Herstellung von Thiazolyl-2-sulfenamiden der allgemeinen Formel I eignen, sind die folgenden Verbindungen:

2-Mercapto-4-methylbenzothiazol
2-Mercapto-5-methylbenzothiazol
2-Mercapto-6-methylbenzothiazol
2-Mercapto-4,5-dimethylbenzothiazol
2-Mercapto-4-phenylbenzothiazol
2-Mercapto-4-methoxybenzothiazol
2-Mercapto-6-methoxybenzothiazol
2-Mercapto-5,6-dimethoxybenzothiazol
2-Mercapto-6-methoxy-4-nitrobenzothiazol
2-Mercapto-6-äthoxybenzothiazol
2-Mercapto-4-chlorbenzothiazol
2-Mercapto-5-chlorbenzothiazol
2-Mercapto-6-chlorbenzothiazol
2-Mercapto-7-chlorbenzothiazol
2-Mercapto-5-chlor-6-methoxybenzothiazol
2-Mercapto-5-chlor-4-nitrobenzothiazol
2-Mercapto-5-chlor-6-nitrobenzothiazol
2-Mercapto-4,5-dichlorbenzothiazol
2-Mercapto-4,7-dichlorbenzothiazol
2-Mercapto-5-nitrobenzothiazol
2-Mercapto-6-nitrobenzothiazol
2-Mercapto-4-phenylbenzothiazol

2-Mercapto-naphthothiazol
2-Mercapto-6-hydroxybenzothiazol

Anstelle der genannten Mercaptothiazole lassen sich als Ausgangsstoffe für das erfindungsgemäße Verfahren ebensogut die entsprechenden Dithiazolyl-2,2'-disulfide einsetzen. Spezielle Beispiele dafür sind die folgenden Verbindungen:

Dibenzthiazolyl-(2,2')-disulfid
Bis-(6-methylbenzthiazolyl-(2))-disulfid
Bis-(4-methylbenzthiazolyl-(2))-disulfid
Bis-(4-methoxybenzthiazolyl-(2))-disulfid
Bis-(6-äthoxybenzthiazolyl-(2))-disulfid
Bis-(5-chlorbenzthiazolyl-(2))-disulfid
Bis-(5-chlor-4-nitrobenzthiazolyl-(2))-disulfid
Bis-(3-chlor-6-nitrobenzthiazolyl-(2))-disulfid
Bis-(6-nitrobenzthiazolyl-(2))-disulfid

Bevorzugt wird Dibenzthiazolyl-(2,2'-disulfid eingesetzt.

Geeignete beim erfindungsgemäßen Verfahren einzusetzende primäre Amine sind beispielsweise n-Propylamin, Isopropylamin, n-Butylamin, sec.-Butylamin, tert.-Butylamin, Pentylamin. Hexylamin, Heptylamin, Octylamin, Decylamin, Cyclopentylamin, Cyclohexylamin, Cycloheptylamin, Cyclooctylamin oder Benzylamin. Geeignete sekundäre Amine sind beispielsweise Diäthylamin, Di-n-propylamin, Diisopropylamin, Di-n-butylamin, Di-tert.-butylamin, Dicyclohexylamin, Pyrrolidin, Piperidin, Piperazin, Morpholin, 2,6-Dimethylmorpholin und 3,5-Dimethylmorpholin. Bevorzugt eingesetzte Amine sind das Cyclohexylamin, tert.-Butylamin und Morpholin. Auf die hier aufgezählten Amine sind jedoch die erfindungsgemäß einsetzbaren Amine nicht beschränkt.

Der erfindungsgemäße Zusatz von Ammoniak hat den überraschenden Effekt, daß die Reaktion auch bei einem Überschuß des als Oxidationsmittel eingesetzten Sauerstoffs auf der Stufe des gewünschten Sulfenamids stehen bleibt. Die Reaktion ist somit leicht regulierbar, und es kommt so vor allem nicht zu Ausbeute-Verlusten bzw. Verunreinigungen des Endprodukts durch Überoxidation (erkennbar auch am erforderlichen hohen Überschuß an Oxidationsmittel) wie beim Arbeiten ohne Ammoniak, beispielsweise im Falle der Vorveröffentlichungen DE-OS-2 349 934 und DE-OS-2 356 686. Außerdem wird durch den Ammoniak eine erhebliche Steigerung der Reaktionsgeschwindigkeit erreicht; in vielen Fällen findet in Abwesenheit von Ammoniak überhaupt keine Reaktion mit dem Sauerstoff statt.

Auf keinen Fall aber entsteht durch den erfindungsgemäßen Zusatz von Ammoniak, auch nicht bei hohem Überschuß, ein Sulfenamid

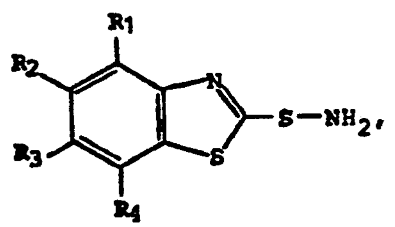

sondern das jeweils gewünschte N-substituierte Sulfenamid in hoher Selektivität. Gemäß der in DE-OS-2 349 934 und DE-OS-2 356 686 gegebenen Lehre soll mit Ammoniak (anstelle eines sekundären oder primären Amins) angeblich ein solches, am Stickstoff unsubstituiertes Sulfenamid gebildet werden; tatsächlich entsteht hierbei aber das entsprechende Dibenzthiazolldisulfid (vgl. DE-OS-3 113 298).

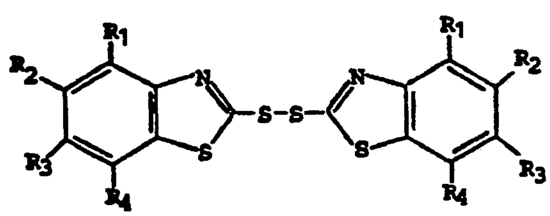

Die Menge des erfindungsgemäß einzusetzenden Ammoniaks kann in weiten Grenzen variiert werden. Schon ein Ammoniak-Anteil von nur 0,2 Gew.-%, bezogen auf das Gewicht des Reaktionsgemisches, zeigt einen vorteilhaften Effekt. Es empfiehlt sich, einen Anteil von 25 Gew.-% Ammoniak nicht zu überschreiten. Es ist besonders vorteilhaft, einen Anteil von 1 bis 15 Gew.-% Ammoniak, bezogen auf das Gewicht des Reaktionsgemischs, einzusetzen.

Die Wahl des Reaktionsmediums ist für das erfindungsgemäße Verfahren zur Erzielung höherer Ausbeuten und Selektivitäten von großer Bedeutung und hängt insbesondere von der Natur des umzusetzenden primären oder sekundären Amins ab.

In einigen Fällen ist es günstig, einen Überschuß des jeweiligen Amins als alleiniges Reaktionsmedium zu verwenden. In vielen anderen Fällen ist es vorteilhaft, ein aus dem jeweiligen Amin und Wasser bestehendes Gemisch alt Reaktionsmedium einzusetzen. In manchen Fällen ist es auch vorteilhaft, die Reaktion allein mit Wasser als Reaktionsmedium durchzuführen.

Der jeweils günstigste Wasseranteil im Reaktionsmedium ist stark abhängig vom jeweils eingesetzten primären oder sekundären Amin (z. B. von seiner Basizität, Reaktivität, Mischbarkeit mit Wasser oder seinen sterischen Eigenschaften) sowie von dem entstehenden Sulfenamid (z. B. von seiner Stabilität unter Reaktionsbedingungen, Löslichkeit) und kann leicht vom Fachmann durch wenige Vorversuche ermittelt werden.

Beispielsweise verwendet man bei der Herstellung von Cyclohexylbenzthiazolylsulfenamid aus Cyclohexylamin und 2-Mercapto-benzthiazol bzw. Dibenzthiazyl-2,2'-disulfid als Reaktionsmedium Cyclohexylamin mit einem Wasseranteil von bevorzugt 20 bis 100 % und besonders bevorzugt von 90 bis 99 % (der stöchiometrische, für die Reaktion notwendige Aminanteil ist dabei nicht mitgerechnet).

Bei der Herstellung von Morpholinyl-benzthiazolylsulfenamid aus 2-Mercaptobenzthiazol bzw. Dibenzthiazyl-2,2'-disulfid und Morpholin wird dagegen Morpholin bevorzugt mit einem Wasseranteil von 0 bis 75 % und besonders bevorzugt 10 bis 50 % als Reaktionsmedium verwendet.

Für die Herstellung von t-Butylbenzthiazolsulfenamid aus t-Butylamin und 2-Mercaptobenzthiazol bzw. Dibenzthiazolyl-2,2'-disulfid wird ein Reaktionsmedium aus t-Butylamin und Wasser mit einem Wassergehalt von 0 bis 85 % bevorzugt. Besonders bevorzugt ist ein Wassergehalt von 0 bis 20 %.

Das Verhältnis von primärem oder sekundärem Amin zu dem 2-Mercaptothiazol bzw. Dithiazolyl-2,2'-disulfid kann entsprechend dem verwendeten Reaktionsmedium ebenfalls in weiten Grenzen variieren, wobei zur Erzielung eines reinen Produktes das Amin mindestens in stöchiometrischer Menge oder im stöchiometrischen Überschuß eingesetzt werden sollte. Im allgemeinen liegt die eingesetzte Aminmenge im Bereich zwischen 1,1 und 15 mol pro 1 mol Mercaptothiazol bzw. 0,5 mol Dithiazolyl-2,2'-disulfid. Der Einsatz höherer Aminmengen wird aus ökonomischen Gründen weniger bevorzugt.

In einzelnen Fällen, z. B. bei zu geringer Mischbarkeit des primären oder sekundären Amins mit Wasser, oder zur Erhöhung der Katalysatorlöslichkeit ist es vorteilhaft, dem Reaktionsgemisch ein organisches Lösungsmittel (z. B. Alkohole) zuzusetzen, dessen Menge der Fachmann durch wenige Vorversuche leicht ermitteln kann. Im allgemeinen wird aber bevorzugt, ohne ein zusätzliches Lösungsmittel zu arbeiten.

Als Katalysator wird beim erfindungsgemäßen Verfahren metallisches Kupfer oder ein Kupferderivat eingesetzt. Das metallische Kupfer kommt vorzugsweise als Kupferpulver zur Anwendung. Als Kupferderivat kommen alle ein- oder zweiwertigen anorganischen, organischen, einfachen oder komplexen Kupfersalze, mit Ausnahme der Phthalocyaninkomplexe in Betracht. Beispiele für geeignete einwertige Kupfersalze sind Kupfer-(I)-chlorid, -bromid und -jodid, Additionsverbindungen dieser Kupfer-(I)-halogenide mit Kohlenmonoxid, komplexe Kupfer-(I)-salze wie die Alkalichlorcuprate, komplexe Ammoniakate des Kupfer-(I)-cyanids, z. B. Cyanocuprate wie Kalium-tricyanocuprat-(I), Doppelsalze mit Kupfer-(I)-rhodanid, Kupfer-(I)-acetat, Kupfer-(I)-sulfit und komplexe Doppelsulfide aus Kupfer-(I)-sulfid und Alkalipolysulfiden. Beispiele geeigneter Kupfer-(II)-salze sind Kupfer-(II)-chlorid, -bromid, -sulfid, -sulfat, -nitrat, -nitrit, -rhodanid, -cyanid, Cu-(II)-Salze von Carbonsäuren wie Kupfer-(II)-acetat sowie die komplexen Ammoniakate von Kupfer-(II)-salzen. Auch Kupfer-(I)-oxid ist sehr gut als Katalysator geeignet. Selbstverständlich können auch Gemische aus mehreren der genannten Katalysatoren eingesetzt werden.

Die erforderliche Menge des Kupferkatalysators ist überraschend gering. Sie liegt vorzugsweise im Bereich von 0,005 bis 5 mmol, bezogen auf 1 Mol Mercaptothiazol bzw. Thiazolyl-disulfid. Es können auch geringere Katalysatormengen zur Anwendung gelangen, jedoch müssen hierbei längere Reaktionszeiten in Kauf genommen werden. Höhere Mengen an Katalysatoren erhöhen zwar die Reaktionsgeschwindigkeit, sind aber nicht empfehlenswert, weil dann die Gefahr besteht, daß der Katalysator das Reaktionsprodukt verunreinigt.

Verfahrenswesentlich ist auch die Reaktionstemperatur. Sie liegt im Bereich von 0 bis 100°C. Bei tieferen Temperaturen ist die Selektivität besonders hoch, nimmt die Reaktionsgeschwindigkeit aber ab, so daß für die Reaktion bis zu mehrere Tage benötigt werden, um zu quantitativen Umsätzen zu gelangen. Es kann allerdings eine niedrige Reaktionstemperatur beispielsweise im Bereich von 0 bis 20°C dann von Vorteil sein, wenn ein sehr reines Endprodukt erwünscht ist, so daß dafür eine längere Verweilzeit des Reaktionsgemischs im Reaktor in Kauf genommen werden kann. Bei höheren Temperaturen erhöht sich die Reaktionsgeschwindigkeit beträchtlich; es tritt jedoch dann schon gleichzeitig durch Überoxidation bzw. Zersetzung des Sulfenamids eine Abnahme der Selektivität ein. Dies gilt vor allem für Temperaturen oberhalb 100°C, in geringerem Umfang auch schon für Temperaturen unterhalb 100°C, jedoch oberhalb 80°C. Eine hohe Reaktionstemperatur im Bereich von 80 bis 100°C wird also dann von Vorteil sein, wenn an die Produkt-Reinheit weniger hohe Anforderungen gestellt werden, dafür aber ein sehr hoher Produkt-Durchsatz im Reaktor erreicht werden kann.

Vorzugsweise wird das erfindungsgemäße Verfahren bei 0 bis 80°C durchgeführt. Ein sehr geeigneter Temperaturbereich sind Verfahrenstemperaturen zwischen 10 und 90°C. Besonders bevorzugt ist ein Temperaturbereich von 20 bis 80°C.

Im allgemeinen wird das erfindungsgemäße Verfahren bei Sauerstoffdrücken bzw. Sauerstoffpartialdrücken von wenigstens 0,1 bar durchgeführt. Wie zu erwarten, erhöht sich mit steigendem Druck die Reaktionsgeschwindigkeit. Aus ökonomischen und sicherheitstechnischen Gründen wird ein Druckbereich von 1 - 10 bar bevorzugt.

Die Reaktionsdauer hängt von den Verfahrensbedingungen sowie von dem eingesetzten primären bzw. sekundären Amin ab. Unter günstigen Bedingungen und bei Einsatz von Mercaptothiazolen beträgt sie wenige Minuten bis 3 Stunden.

Bei Einsatz von Dithiazoyldisulfiden sind die Reaktionszeiten kürzer, da in diesen Fällen für die Oxidation nur die halbe Sauerstoffmenge benötigt wird.

Die Durchführung des erfindungsgemäßen Verfahrens erfolgt in einfacher Weise dadurch, daß der Sauerstoff bzw. das sauerstoffhaltige Gas unter den angegebenen Druck- und Temperaturbedingungen auf das Reaktionsgemisch aufgepreßt oder in bzw. durch das Reaktionsgemisch geleitet wird, das aus primärem oder sekundärem Amin, Mercaptothiazol bzw. Dithiazolyl-2,2'-disulfid, Kupferkatalysator, Ammoniak und gegebenenfalls Wasser und/oder mit Wasser mischbarem Lösungsmittel besteht.

Das Mercaptothiazol bzw. Dithiazolyl-2,2'-disulfid und/oder das primäre oder sekundäre Amin können auch während der Reaktion dem Reaktionsgemisch zugeführt werden.

In den meisten Fällen fällt das gewünschte Endprodukt bereits während der Reaktion oder am Reaktionsende nach Abkühlen in fester Form aus dem Reaktionsgemisch aus und kann abfiltriert werden. In anderen Fällen erhält man das Produkt durch Verdünnen mit Wasser oder Einengen des Reaktionsgemisches. Flüssige Produkte werden durch destillative oder extraktive Aufarbeitung in reiner Form erhalten.

Bei der technischen Durchführung des erfindungsgemäßen Verfahrens ist es vorteilhaft, die Mutterlauge im Kreislauf zu führen.

Nach dem Abfiltrieren des Sulfenamids kann die Mutterlauge an 2-Mercaptothiazol bzw. Dithiazolyl-2,2'-disulfid aufgefrischt und direkt und praktisch beliebig oft wieder eingesetzt werden, ohne daß die Selektivität und die Ausbeute nachteilig beeinflußt werden. Das Verfahren eignet sich daher vorzüglich für eine kontinuierliche Arbeitsweise.

Das erfindungsgemäße Verfahren zeichnet sich gegenüber dem Stand der Technik vor allem durch die folgenden Vorteile aus:

Es konnten verbesserte Produktausbeuten (in den meisten Fällen über 95 % d. Th.) und hohe Selektivitäten (zwischen 95 und 100 %) erzielt werden. Die Produkte fallen in hoher Reinheit an, und können daher in der Regel ohne weitere Reinigung ihrer Bestimmung zugeführt werden. Wegen der leichten Durchführbarkeit, der erhöhten Reaktionsgeschwindigkeit, der Wiedereinsetzbarkeit der Mutterlauge und der Möglichkeit kontinuierlicher Arbeitsweise wird somit ein sehr wirtschaftliches Verfahren zur Verfügung gestellt.

Das Verfahren wird durch die nachstehenden Ausführungsbeispiele näher erläutert.

**Beispiel 1**

In einem Glasautoklaven, der mit einem Doppelmantel zur Zirkulation einer Heizflüssigkeit, einem Thermometer, einem Druckgerät und einer Rührvorrichtung ausgerüstet ist, werden 25 g (0,15 mol) 2-Mercaptobenzthiazol, 6 mg (0,03 mmol) $Cu(OAc)_2 \cdot H_2O$, 170 g Wasser und 10,25 g (0,6 mol) Ammoniak vorgelegt. Das Reaktionsgemisch wird auf 50°C erwärmt, intensiv gerührt und mit 4,1 bar Sauerstoff beaufschlagt, wobei gleichzeitig 18,1 g (0,183 mol) Cyclohexylamin während 3 Stunden dem Reaktionsgemisch zugeführt wird. Sofort wird eine Sauerstoffaufnahme registriert, und es bildet sich ein weißer Niederschlag. Nach 5 Stunden ist die Sauerstoffaufnahme nur noch gering; die Reaktion wird beendet. Der weiße Niederschlag wird abfiltriert, mit Ammoniaklösung und Wasser gewaschen und getrocknet. So werden 38,1 g eines Produktes erhalten, das in seinen analytischen Daten (Elementaranalyse, IR, $^1$HNMR, MS) mit N-Cyclo-hexylbenzthiazolsulfenamid identisch ist, und dessen Reinheit durch Hochdruckflüssigkeitschromatographie-Analyse zu 98 % bestimmt wird (Fp. 102°C). Die Mutterlauge enthält noch 0,7 g nicht umgesetztes Mercaptobenzthiazol. Demnach beträgt der Umsatz 97,2 %, die Ausbeute an Cyclohexylbenzthiazolsulfenamid 96,2 %. d. Th. (Selektivität 99 %).

**Beispiel 2 (Vergleichsbeispiel)**

Es wird wie in Beispiel 1 gearbeitet, aber ohne Zusatz von Ammoniak. Unter diesen Bedingungen erfolgt praktisch keine Sauerstoffaufnahme. Nach 5 Stunden wird das eingesetzte Mercaptobenzthiazol im wesentlichen unverändert bzw. in Form seines Cyclohexylammoniumsalzes zurückgewonnen.

**Beispiel 3**

Das Verfahren wurde gemäß Beispiel 1 durchgeführt; aber anstelle von 2-Mercaptobenzthiazol wurden 25 g (0,075 mol) 2.2'-Dibenzthiazolyldisulfid eingesetzt. Außerdem wurde das Cyclohexylamin in 1,5 Stunden zugegeben. Die Sauerstoffaufnahme war nach 3,5 Stunden nur noch gering; die Reaktion wurde beendet. Die Ausbeute an N-Cyclohexylbenzthiazolsulfenamid betrug 38,6 g, entsprechend 97,4 % d. Th. (Produktreinheit 98 %).

**Beispiel 4**

20 g (0,12 mol) 2-Mercaptobenzthiazol, 12 mg $Cu(OAc)_2 \cdot H_2O$ (0,06 mmol), 170 g Wasser und 10,25 g (0,6 mol) Ammoniak werden in der in Beispiel 1 beschriebenen Weise mit Sauerstoff zur Reaktion gebracht, wobei 13,2 g (0,132 mol) Cyclohexylamin während 3 Stunden zugegeben werden. Die Reaktionstemperatur beträgt 60°C, der Sauerstoffdruck 4 bar. Nach 3,5-stündiger Reaktionszeit wird der Mercaptobenzthiazol-Umsatz zu 98,1 % bestimmt; die Ausbeute an Cyclohexylbenzthiazolsulfenamid beträgt 96,2 % d. Th.

**Beispiel 5**

In der in Beispiel 1 beschriebenen Reaktionsapparatur wird ein Reaktionsgemisch, bestehend aus 16,7 g Mercaptobenzthiazol (0,1 mol), 49,6 g Cyclohexylamin (0,5 mol), 51 g Wasser, 1,7 g Ammoniak (0,1 mol) und 60 mg $Cu(OAc)_2 \cdot H_2O$ (0,3 mmol) mit Sauerstoff zur Reaktion gebracht. Der Sauerstoffdruck beträgt 4 bar, die Reaktionstemperatur 40°C und die Reaktionszeit 2 Stunden. N-Cyclohexylbenzthiazolsulfenamid fällt als weißer, kristalliner Niederschlag an, in einer Ausbeute von 24,3 g entsprechend 92 % d. Th. Der Mercaptobenzthiazol-Umsatz wird durch Hochdruckflüssigkeitschromatographie-Analyse zu 95,9 % bestimmt.

**Beispiel 6**

In der in Beispiel 1 beschriebenen Reaktionsapparatur werden 33,4 g Mercaptobenzthiazol (0,2 Mol), 111,3 g t-Butylamin (1,52 mol), 3 g Wasser, 3,25 g Ammoniak (0,19 mol) und 200 mg $Cu(OAc)_2 \cdot H_2O$ (1 mmol) vorgelegt. Das Reaktionsgemisch wird auf 60°C erhitzt, intensiv gerührt und mit 3 bar Sauerstoff beaufschlagt. Sofort wird eine Sauerstoffaufnahme registriert, die nach 70 min. zum Stillstand kommt: Die Raktion ist beendet. Beim Abkühlen kristallisiert aus der Mutterlauge ein weisser Feststoff (32,6 g) der abfiltriert, gewaschen und getrocknet wird und in seinen analytischen Daten (Elementaranalyse, MS, IR, $^1$HNMR) mit N-t-Butylbenzthiazolsulfenamid identisch ist. Die Reinheit wurde durch Hochdruckflüssigkeitschromatographie-Analyse zu 98,4 % ermittelt (Fp. 108 - 110°C). Die Mutterlauge enthält weitere 14,9 g des Produktes, die z. B. durch Verdünnen mit Wasser ausgefällt und isoliert werden könne. Demnach beträgt die Gesamtausbeute an t-Butylbenzthiazol-sulfenamid 47,5 g entsprechend 99,8 % d. Th.

**Beispiel 7**

Wie in Beispiel 6 beschrieben, werden 33,2 g Dibenzthiazolyl-2,2'-disulfid (0,1 mol) 111,3 g t-Butylamin (1,52 mol), 5,7 g Wasser, 3,25 g Ammoniak (0,19 mol) und 200 mg $Cu(OAc)_2 \cdot H_2O$ (1 mmol) mit Sauerstoff zur Reaktion gebracht. Die Reaktionstemperatur beträgt 40°C, der Sauerstoffdruck 3,5 bar. Nach 75 min. ist die Reaktion beendet (keine weitere Stoffaufnahme); die Ausbeute an t-Butylbenzthiazolsulfenamid beträgt 99,9 % d. Th. (t-Butylbenzthiazolsulfenamid-Gehalt: 98,9 %).

**Beispiel 8**

In dem folgenden Beispiel wird ohne Zusatz von Wasser gearbeitet. Es wird in der in Beispiel 6 beschriebenen Weise verfahren. Das Reaktionsgemisch besteht aus 33,4 g Mercaptobenzthiazol (0,2 mol), 114,3 g t-Butylamin (1,56 mol), 3,25 g Ammoniak (0,19 mol) und 200 mg $Cu(OAc)_2 \cdot H_2O$ wird bei 40°C mit Sauererstoff (Sauerstoffdruck 3,5 bar) zur Reaktion gebracht. Die Reaktionszeit beträgt 150 min. Das Reaktionsgemisch enthält laut Hochdruckflüssigkeitschromatographie-Analyse 45,9 g t-Butylbenzthiazolsulfenamid (Ausbeute 96,4 % d. Th.), das z. B. durch Abkühlen und Verdünnen mit Wasser ausgefällt und isoliert werden kann. Das Reaktionsgemisch enthält außerdem noch 0,57 g nicht umgesetztes Mercaptobenzthiazol. Umsatz an Mercaptobenzthiazol 98,3 %, Selektivität 98 %.

**Beispiel 9**

Wie in Beispiel 6 beschrieben, werden 33,4 g Mercaptobenzthiazol (0,2 mol), 45,7 g t-Butylamin (0,63 mol), 3,25 g Ammoniak (0,19 mol), 200 mg $Cu(OAc)_2 \cdot H_2O$ (1 mmol) und 60 g Wasser mit Sauerstoff zur Reaktion gebracht. Die Reaktionstemperatur beträgt 40°C, der Sauerstoffdruck 4 bar und die Reaktionszeit 150 min. Die Ausbeute an t-Butylbenzthiazolsulfenamid beträgt 90,8 % d. Th., der Mercaptobenzthiazolumsatz 93,6 % (Selektivität: 97 %).

**Beispiel 10**

Das folgende Beispiel wird bei Raumtemperatur durchgeführt. Wie in Beispiel 6 beschrieben, werden 33,4 g Mercaptobenzthiazol (0,2 mol), 111,3 g t-Butylamin (1,52 mol), 3,0 g Wasser, 3,25 g Ammoniak (0,19 mol) und 200 mg $Cu(OAc)_2 \cdot H_2O$ (1 mmol) mit Sauerstoff zur Reaktion gebracht. Die Reaktionstemperatur beträgt 25°C, der Sauerstoffdruck 4 bar. Nach 6 Stunden ist die Reaktion beendet (keine weitere Sauerstoffaufnahme). Die Ausbeute an t-Butylbenzthiazolsulfenamid beträgt 97,2 % d. Th.

**Beispiel 11**

Im folgenden Beispiel wird als sauerstoffhaltiges Gas Luft verwendet. Wie in Beispiel 6 beschrieben, werden 33,4 g Mercaptobenzthiazol (0,2 mol), 108,6 g t-Butylamin (1,49 mol), 3,25 g Ammoniak (0,19 mol), 5,7 g Wasser und 200 mg $Cu(OAc)_2 \cdot H_2O$ (1 mmol) bei 40°C mit 5 bar Luft beaufschlagt und zur Reaktion gebracht. Nach 180 min. Reaktionszeit beträgt die Ausbeute an t-Butylbenzthiazolsulfenamid 95,1 % d. Th., der Mercaptobenzthiazol-Umsatz 95,5 % (Selektivität 99,6 %).

**Beispiele 12 - 15**

In den folgenden Beispielen kommen unterschiedliche Kupferkatalysatoren zum Einsatz. Es wird wie in Beispiel 6 beschrieben gearbeitet, wobei jeweils 33,4 g Mercaptobenzthiazol (0,2 mol), 108,6 g t-Butylamin (1,49 mol) und 5,7 g Wasser eingesetzt werden. Der Sauerstoffdruck beträgt jeweils 3,5 bar. Die weiteren Reaktionsbedingungen sowie Mercaptobenzthiazol-Umsatz und Ausbeute an t-Butylbenzthiazolsulfenamid sind in der folgenden Tabelle angegeben.

| Beispiel | Katalysator | (mmol) | $NH_3$ (mol) | R-Temp. (°C) | R-Zeit (min.) | Umsatz (%) | Ausbeute (% d. Th.) |
|---|---|---|---|---|---|---|---|
| 12 | $CuSO_4$ | (0,25) | 0,19 | 50 | 240 | 97,8 | 93,9 |
| 13 | CuCl | (1,0) | 0,05 | 60 | 220 | 99,4 | 98,9 |
| 14 | CuO | (0,5) | 0,19 | 60 | 190 | 96,9 | 94,6 |
| 15 | Cu-Pulver | (1,0) | 0,19 | 40 | 150 | 98,5 | 97,0 |

**Beispiel 16**

Zur Herstellung von N-Cyclopentamethylenbenzthiazolsulfenamid wird ein Reaktionsgemisch aus 24,6 g Mercaptobenzthiazol (0,147 mol), 15,6 g Piperidin (0,183 mol), 200 g Wasser, 20,5 g Ammoniak (1,2 mol) und 36 mg $Cu(OAc)_2 \cdot H_2O$ (0,18 mmol) in der in Beispiel 1 beschriebenen Weise mit Sauerstoff zur Reaktion gebracht. Die Reaktionstemperatur beträgt 40°C, der Sauerstoffdruck 4 bar. Nach 5 Stunden ist die Sauerstoffaufnahme nur noch gering, die Reaktion wird abgestellt. Der gebildete Produkt-Niederschlag wird abfiltriert, mit verdünnter, wäßriger Ammoniaklösung und Wasser gewaschen und getrocknet; er enthält laut Hochdruckflüssigkeitschromatographie-Analyse 98 % N-Cyclopentamethylenbenzthiazolsulfenamid. Die Produktausbeute beträgt 93,7 % d. Th. Die Mutterlauge enthält noch 1,03 g nicht umgesetztes Mercaptobenzthiazol (Selektivität 98 %).

**Beispiel 17**

Zur Herstellung von N-Isopropylbenzthiazolsulfenamid werden 31,9 g Mercaptobenzthiazol (0,191 mol), 108,6 g iso-Propylamin (1,83 mol), 1,64 g Ammoniak (0,09 mol), 5,7 g Wasser und 100 mg $Cu(OAc)_2 \cdot H_2O$ (0,5 mmol) bei 40°C mit Sauerstoff zur Reaktion gebracht (Sauerstoffdruck 3 bar). Die Durchführung erfolgt in der in Beispiel 8 beschriebenen Weise. Das Produkt (Fp. 94°C) wird in einer Ausbeute von 90,4 % d. Th. erhalten.

**Beispiel 18**

Zur Herstellung von Morpholino-thiobenzthiazol wird in der in Beispiel 1 beschriebenen Reaktionsapparatur ein Reaktionsgemisch, bestehend aus 25,1 g Mercaptobenzthiazol (0,15 mol), 52,3 g Morpholin (0,6 mol), 5,1 g Ammoniak (0,3 mol), 100 mg $Cu(OAc)_2 \cdot H_2O$ (0,5 mmol) und 36 ml Wasser mit Sauerstoff zur Reaktion gebracht. Der Sauerstoffdruck beträgt 3,8 bar, die Reaktionstemperatur 50°C. Nach 2 Stunden wird die Reaktion beendet (nur noch geringe Sauerstoffaufnahme), der gebildete Niederschlag wird abfiltriert, gewaschen und getrocknet und entspricht in seinen analytischen Werten (Elementaranalyse, MS, IR, $^1$H-NMR) dem Morpholinothiobenzthiazol (Reinheit 98,2 %, Fp. 82 - 84°C). Die Produktausbeute beträgt 35,6 g, entsprechend 94,2 % d. Th.. Die Mutterlauge enthält noch 0,7 g nicht umgesetztes Mercaptobenzthiazol (Selektivität 97 %).

**Beispiel 19**

24,9 g Dibenzthiazolyl-2,2'-disulfid (0,075 mol) werden mit 52,3 g Morpholin (0,6 mol), 2,6 g Ammoniak (0,15 mol), 100

mg $Cu(OAc)_2 \cdot H_2O$ (0,5 mmol) und 27 ml Wasser in der in Beispiel 18 beschriebenen Weise mit Sauerstoff zur Reaktion gebracht. Der Sauerstoffdruck beträgt 4,0 bar, die Reaktionstemperatur 50°C und die Reaktionszeit 140 min. Morpholinothiobenzthiazol wird dabei in einer Ausbeute von 36,1 g erhalten, entsprechend 95,5 % d. Th..

**Beispiel 20 - 22**

Diese Beispiele beschreiben die Herstellung von Cyclohexylbenzthiazolsulfenamid, wobei jeweils Alkohole als zusätzliche Lösungsmittel eingesetzt werden. Die Durchführung erfolgt in der in Beispiel 1 beschriebenen Weise; der Sauerstoffdruck beträgt 4,1 bar. Die weiteren Reaktionsbedingungen, sowie der Umsatz bezüglich Mercaptobenzthiazol und die Ausbeute an Cyclohexylbenzthiazolsulfenamid sind in der folgenden Tabelle zusammengestellt.

| Beispiel | Mercaptobenzthiazol (mol) | Cyclohexylamin (mol) | Wasser (g) | Alkohol (g) | $NH_3$ (mol) | $Cu(OAc)_2$ (mmol) | Reaktions-temp. (°C) | Reaktions-zeit (min.) | Umzatz (%) | Ausbeute (% d. Th.) |
|---|---|---|---|---|---|---|---|---|---|---|
| 20 | 0,20 | 0,22 | 132 | Methanol 36 | 0,8 | 0,06 | 50 | 210 | 97,3 | 94,2 |
| 21 | 0,15 | 0,18 | 114 | Methanol 54 | 0,6 | 0,08 | 50 | 150 | 96,1 | 92,8 |
| 22 | 0,15 | 0,18 | 135 | Ethanol 15 | 0,5 | 0,04 | 60 | 190 | 97,0 | 93,5 |

**Beispiel 23**

Im folgenden Beispiel wird die Reaktion bei 80°C durchgeführt. 50 g (0,30 mol) 2-Mercaptobenzthiazol. 12 mg $Cu(OAc)_2 \cdot H_2O$ (0,06 mmol), 340 g Wasser und 20,4 g (1,2 mol) Ammoniak werden in der in Beispiel 1 beschriebenen Weise mit Sauerstoff zur Reaktion gebracht, wobei 34,0 g (0,343 mol) Cyclohexylamin während 3 Stunden zugegeben werden. Die Reaktionstemperatur beträgt 80°C, der Sauerstoffdruck 4 bar. Nach 3 Stunden ist die Reaktion beendet (keine weitere Sauerstoffaufnahme). Die Ausbeute an N-Cyclohexylbenzthiazolsulfenamid beträgt 85,5 % d. Th.

Die Beispiele 24 bis 26 zeigen, daß mit Ammoniak in Abwesenheit eines primären oder sekundären Amins kein Benzthiazolsulfenamid, sondern Dibenzthiazolyldisulfid gebildet wird.

**Beispiel 24 (Vergleichsbeispiel)**

In der in Beispiel 1 beschriebenen Reaktionsapparatur werden 50 g (0,3 mol) 2-Mercaptobenzthiazol, 12 mg (0,06 mmol) $Cu(OAc)_2 \cdot H_2O$, 20,4 g (1,2 mol) Ammoniak und 320 g Wasser eingebracht. Das Reaktionsgemisch wird auf 50°C erwärmt, wobei eine klare Lösung entsteht, intensiv gerührt und mit einem Sauerstoffdruck von 4 bar beaufschlagt. Sofort wird eine Sauerstoffaufnahme registriert, und es entsteht ein Niederschlag infolge Bildung von Dibenzthiazolyldisulfid. Nach 1 Stunde wird der Versuch abgeschaltet, der Niederschlag wird abfiltriert, mit wäßrigem Ammoniak sowie mit Wasser gewaschen und im Vakuum bei 70°C getrocknet. So werden 47,7 g eines Produktes erhalten, das in reinen analytischen Daten (Elementaranalyse, IR, $^1$H-MMR und MS) mit Dibenzthiazolyldisulfid identisch ist und dessen Reinheit durch chromatographische Analyse zu 100 % bestimmt wird (Fp. 178°C).

Die Mutterlauge enthält laut gaschromatographischer Analyse 1,8 g nicht umgesetztes 2-Mercaptobenzthiazol. Demnach beträgt der 2-Mercaptobenzthiazol-Umsatz 96,4 %, die Dibenzthiazolyldisulfid-Ausbeute 96,0 % d. Th. (Selektivität 99,6 %).

**Beispiel 25 (Vergleichsbeispiel)**

In der in Beispiel 24 beschriebenen Weise werden 50 g (0,3 mol) 2-Mercaptobenzthiazol, 8 mg (0,04 mmol) $Cu(OAc)_2 \cdot H_2O$, 40,8 g (2,4 mol) Ammoniak und 300 g Wasser auf 50°C erwärmt, intensiv gerührt und mit einem Sauerstoffdruck von 4 bar beaufschlagt, wobei sofort Sauerstoffaufnahme registriert und ein Niederschlag von Dibenzthiazolyldisulfid gebildet wird. Nach einer Reaktionszeit von 2,5 Stunden betrug der Umsatz an 2-Mercaptobenzthiazol 95,4 % und die Ausbeute an Dibenzthiazolyldisulfid 94,1 % d. Th. (Selektivität: 98,6 %).

**Beispiel 26 (Vergleichsbeispiel)**

In der in Beispiel 1 beschriebenen Reaktionsapparatur werden 40 g (0,24 mol) 2-Mercaptobenzthiazol, 12,24 g (0,72 mol) Ammoniak und 120 g Isopropanol eingebracht. Das Reaktionsgemisch wird auf 70°C erwärmt, kräftig gerührt und mit einem Sauerstoffdruck von 4 bar beaufschlagt. Sofort wird eine Sauerstoffaufnahme registriert und es entsteht ein Niederschlag infolge Bildung von Dibenzthiazolyldisulfid.

Nach 6,5 Stunden wird der Versuch abgestellt, der Niederschlag wird abfiltriert, mit Isopropanol gewaschen und im Vakuum bei 50°C getrocknet. So werden 37,8 g eines Produktes erhalten, das in reinen analytischen Daten (Elementaranalyse, IR, NMR und MS) mit Dibenzthiazolyldisulfid übereinstimmt und dessen Reinheit durch

chromatographische Analyse zu 100 % bestimmt wird (Fp. 178°C).

Aus der Mutterlauge werden nach Einengen durch extraktive Trennung mit Methanol weitere 0,5 g Dibenzthiazolyldusulfid isoliert. Weiterhin wird die Menge des nicht umgesetzten 2-Mercaptobenzthiazols im Rückstand durch potentiometrische Titration mit wäßriger Silbernitratlösung zu 0,9 g bestimmt. Demnach beträgt die Ausbeute an Dibenzthiazolyldisulfid 96,4 % d. Th. bei einem Mercaptobenzthiazolumsatz von 97,8 %.

**Patentansprüche**

1. Verfahren zur Herstellung von Benzthiazolylsulfenamiden der allgemeinen Formel

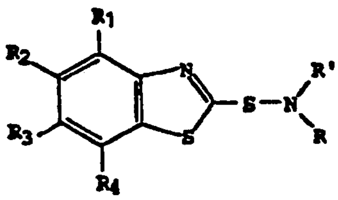

(I)

in der

$R_1$, $R_2$, $R_3$ und $R_4$ gleich oder verschieden sein können und jeweils für Wasserstoff, Chlor, eine Nitrogruppe, eine Hydroxylgruppe, einen Alkyl- oder Alkoxylrest mit 1 bis 6 Kohlenstoffatomen, einen Cycloalkyl- oder Arylrest mit 6 bis 12 Kohlenstoffatomen oder für andere inerte Substituenten stehen, und in der

R und R' gleich oder verschieden sein können und für eine geradkettige, verzweigte oder cyclische Alkylgruppe stehen, wobei R und R' über ein Heteroatom und/oder Kohlenstoffatome miteinander verbunden sein können, so daß sie mit dem Stickstoff eine heterocyclische Gruppierung mit einem oder mehreren Heteroatomen bilden, oder in der R für einen Wasserstoffrest und R' für eine geradkettige verzweigte oder cyclische Alkylgruppe mit 3 bis 8 Kohlenstoffatomen stehen,

durch Umsetzen eines 2-Mercaptobenzthiazols oder Dibenzthiazolyl-2,2'-disulfids der allgemeinen Formel

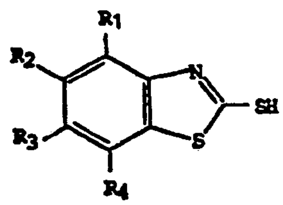

(II)

bzw.

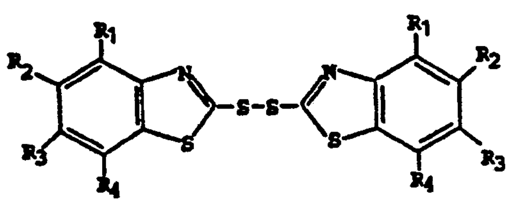

(III)

in der $R_1$, $R_2$, $R_3$ und $R_4$ die oben angegebene Bedeutung haben, mit einem primären oder sekundären Amin der allgemeinen Formel

R
|
HN
|
R'

(IV)

in der R und R' ebenfalls die oben angegebene Bedeutung haben, in Gegenwart von Sauerstoff oder eines sauerstoffhaltigen Gases und von Kupfer oder einem Kupferderivat mit Ausnahme der Phthalocyaninkomplexe bei Temperaturen von 0 bis 100°C, dadurch gekennzeichnet, daß man die Umsetzung in Gegenwart von Ammoniak durchführt und als Reaktionsmedium Wasser oder einen Überschuß des oben genannten primären oder sekundären Amins oder eine Mischung dieses überschüssigen Amins mit Wasser und/oder mit einem wassermischbaren organischen Lösungsmittel einsetzt.

2. Verfahren nach Anspruch 1, dadurch gekennzeichnet, daß man 0,2 bis 25 Gew.-% Ammoniak, bezogen auf das Gewicht des Reaktionsgemischs, einsetzt.

3. Verfahren nach den Ansprüchen 1 und 2, dadurch gekennzeichnet, daß man 1 bis 15 Gew.-% Ammoniak, bezogen auf das Gewicht des Reaktionsgemischs, einsetzt.

4. Verfahren nach den Ansprüchen 1 bis 3, dadurch gekennzeichnet, daß man die Umsetzung mit einem 2-Mercaptobenzthiazol oder einem Dibenzthiazolyl-2,2'-disulfid ausführt, in dem $R_1$, $R_2$, $R_3$ und $R_4$ der Formeln (II) und (III) jeweils für Wasserstoff steht.

5. Verfahren nach den Ansprüchen 1 bis 4, dadurch gekennzeichnet, daß man die Umsetzung bei Temperaturen von 0 bis 80°C durchführt.

6. Verfahren nach den Ansprüchen 1 bis 5, dadurch gekennzeichnet, daß man die Umsetzung bei Temperaturen von 20 bis 80°C durchführt.

7. Verfahren nach den Ansprüchen 1 bis 6, dadurch gekennzeichnet, daß man die Umsetzung in Gegenwart von 0,005 bis 5 mmol Kupferkatalysator, bezogen auf 1 val eines 2-Mercaptobenzthiazols bzw. Dibenzthiazolyl-2,2'-disulfids, durchführt.

8. Verfahren nach den Ansprüchen 1 bis 7, dadurch gekennzeichnet, daß man ein 2-Mercaptobenzthiazol oder ein Dibenzthiazolyl-2,2'-disulfid mit Cyclohexylamin, tert.-Butylamin oder Morpholin umsetzt.

**Claims**

1. Process for the preparation of benzothiazolylsulfenamides of the general formula

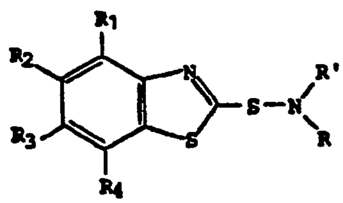

(I)

in which
$R_1$, $R_2$, $R_3$ and $R_4$ can be identical or different and each stand for hydrogen, chlorine, a nitro group, a hydroxy group, an alkyl or alkoxyl [sic] radical having 1 to 6 carbon atoms, a cycloalkyl or aryl radical having 6 to 12 carbon atoms or for other inert substituents, and in which
R and R' can be identical or different and stand for a straight-chain, branched or cyclic alkyl group, it being possible for R and R' to be connected to one another via a heteroatom and/or carbon atoms, thus forming together with the nitrogen a heterocyclic grouping which contains one or more heteroatoms, or in which R stands for a hydrogen radical and R' for a straightchain, branched or cyclic alkyl group having 3 to 8 carbon atoms
by reaction of a 2-mercaptobenzothiazole or a dibenzothiazolyl 2,2'-disulfide of the general formula

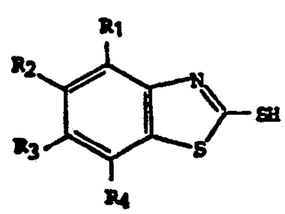

(II)

or

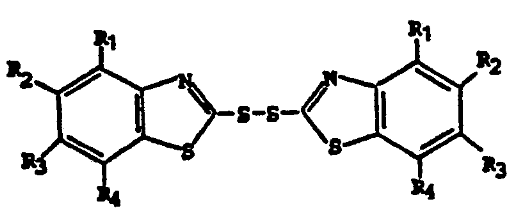

(III)

in which $R_1$, $R_2$, $R_3$ and $R_4$ have the abovementioned meaning with a primary or secondary amine of the general formula

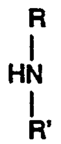

(IV)

in which R and R' also have the above mentioned meaning in the presence of oxygen or an oxygen-containing gas and of copper or a copper derivative with the exception of phthalocyanine complexes at temperatures of 0 to 100°C, characterized in that the reaction is carried out in the presence of ammonia, and that water or an excess of the abovementioned primary or secondary amine or a mixture of this excess amine with water and/or with a watermiscible organic solvent is used as the reaction medium.

2. Process according to Claim 1, characterized in that 0.2 to 25 % by weight of ammonia, relative to the weight of the reaction mixture, are used.

3. Process according to Claims 1 and 2, characterized in that 1 to 15 % by weight of ammonia, relative to the weight of the reaction mixture, are used.

4. Process according to Claims 1 to 3, characterized in that the reaction is carried out using a 2-mercapto-benzothiazole or a dibenzothiazolyl 2,2'-disulfide in which $R_1$, $R_2$, $R_3$ and $R_4$ of the formulae (II) and (III) each stand for hydrogen.

5. Process according to Claims 1 to 4, characterized in that the reaction is carried out at temperatures of 0 to 80°C.

6. Process according to Claims 1 to 5, characterized in that the reaction is carried out at temperatures of 20 to 80°C.

7. Process according to Claims 1 to 6, characterized in that the reaction is carried out in the presence of 0.005 to 5 mmol of a copper catalyst, relative to one equivalent of a 2-mercaptobenzothiazole or dibenzothiazolyl 2,2'-disulfide.

8. Process according to Claims 1 to 7, characterized in that a 2-mercaptobenzothiazole or a dibenzothiazolyl 2, 2'-disulfide is reacted with cyclohexylamine, tert.-butylamine or morpholine.

**Revendications**

1. Procédé de préparation de benzothiazolylsulfénamides de formule générale:

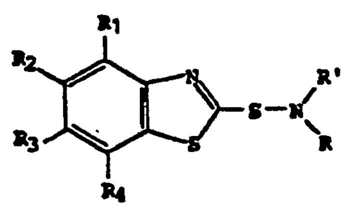

(I)

dans laquelle
$R_1$, $R_2$, $R_3$ et $R_4$ peuvent être identiques ou différents et représentent chacun un atome d'hydrogène ou de chlore ou un groupe nitro, hydroxyle, alkyle ou alcoxy comportant 1 à 6 atomes de carbone, ou cycloalkyle ou aryle comportant 6 à 12 atomes de carbone, ou d'autres substituants inertes, et dans laquelle
R et R' peuvent être identiques ou différents et représentent un groupe alkyle à chaîne droite, ramifiée ou cyclique, R et R' pouvant être liés l'un à l'autre par l'intermédiaire d'un hétéroatome et/ou d'atome de carbone, de manière à former avec l'atome d'azote un groupement hétérocyclique comportant un ou plusieurs hétéroatomes, ou bien dans laquelle R représente un atome d'hydrogène et R' représente un groupe alkyle à chaîne droite, ramifiée ou cyclique, comportant de 3 à 8 atomes de carbone,
par réaction d'un 2-mercaptobenzothiazole ou d'un dibenzothiazolyl-2,2'-disulfure, de formules générales respectives

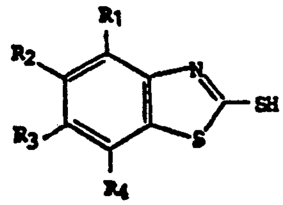

(II)

et

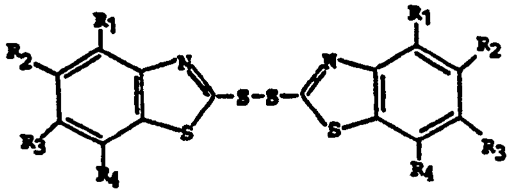

(III)

dans lesquelles $R_1$, $R_2$, $R_3$ et $R_4$ ont les significations indiquées ci-dessus, avec une amine primaire ou secondaire de formule générale

$$HN(R)(R')$$

(IV)

dans laquelle R et R' ont également les significations indiquées ci-dessus, en présence d'oxygène ou d'un gaz contenant de l'oxygène et de cuivre ou d'un dérivé de cuivre, à l'exception des complexes de phtalocyanine, à des températures de 0 à 100°C, le procédé étant caractérisé en ce que l'on effectue la réaction en présence d'ammoniac et en ce que l'on utilise, comme milieu réactionnel, de l'eau ou un excès de l'amine primaire ou secondaire mentionnée ci-dessus ou un mélange de cette amine en excès avec de l'eau et/ou avec un solvant organique miscible à l'eau.

2. Procédé selon la revendication 1, caractérisé en ce que l'on utilise de 0,2 à 25 % en poids d'ammoniac, par rapport au poids du mélange réactionnel.

3. Procédé selon les revendication 1 et 2, caractérisé en ce que l'on utilise de 1 à 15 % en poids d'ammoniac, par rapport au poids du mélange réactionnel.

4. Procédé selon les revendication 1 à 3, caractérisé en ce que l'on effectue la réaction avec un 2-mercaptobenzothiazole ou un dibenzothiazolyl-2,2'-disulfure, dans lequel $R_1$, $R_2$, $R_3$ et $R_4$, dans les formules (II) et (III), représentent chacun un atome d'hydrogène.

5. Procédé selon les revendications 1 à 4, caractérisé en ce que l'on effectue la réaction à des températures de 0 à 80°C.

6. Procédé selon les revendications 1 à 5, caractérisé en ce que l'on effectue la réaction à des températures de 20 à 80°C.

7. Procédé selon les revendications 1 à 6, caractérisé en ce que l'on effectue la réaction en présence de 0,005 à 5 moles de catalyseur au cuivre, pour un équivalent de 2-mercaptobenzothiazole ou de dibenzothiazolyl-2,2'-disulfure.

8. Procédé selon les revendications 1 à 7, caractérisé en ce que l'on fait réagir un 2-mercaptobenzothiazole ou un dibenzothiazolyl-2,2'-disulfure avec de la cyclohexylamine, de la tert.-butylamine ou de la morpholine.